# FASCICULE DE BREVET EUROPEEN

(11) **EP 0 939 649 B1**
(45) Date de publication et mention de la délivrance du brevet: **03.04.2002**
(21) Numéro de dépôt: 97909390.3
(22) Date de dépôt: 10.10.1997
(51) Int. Cl.: A61K 39/39

(54) **ADJUVANT, NOTAMMENT SOUS FORME D'UNE EMULSION CONTENANT UN CATION METALLIQUE TRIVALENT ET COMPOSITION VACCINALE LE COMPRENANT**
ADJUVANS, INSBESONDERE IN FORM EINER DREIWERTIGEN METALLKATION-EMULSION SOWIE DIESE ENTHALTENDE IMPFSTOFFZUSAMMENSETZUNG
ADDITIVE, IN PARTICULAR IN THE FORM OF AN EMULSION CONTAINING A TRIVALENT METAL CATION AND VACCINE COMPOSITION CONTAINING SAME

(30) Priorité: 18.10.1996 FR 9612718
(43) Date de publication de la demande: 08.09.1999
(73) Titulaire: SOCIETE D'EXPLOITATION DE PRODUITS POUR LES INDUSTRIES CHIMIQUES, S.E.P.P.I.C., 75321 Paris Cédex 07 (FR)
(72) Inventeur: GANNE, Vincent, F-94210 La Varenne Saint Hilaire (FR); AUCOUTURIER, Jérôme, F-92290 Châtenay Malabry (FR)
(74) Mandataire: Conan, Philippe Claude
(86) Numéro de dépôt international: FR9701816
(87) Numéro de publication internationale: WO9817311

(56) Documents cités:
- WO-A-94/16681
- WO-A-95/25542
- WO-A-96/22109
- WO-A-96/32964

## Description

La présente invention concerne de nouveaux adjuvants pour compositions vaccinales ainsi que des compositions comprenant au moins un antigène, notamment un antigène d'origine virale, bactérienne ou parasitaire et au moins un adjuvant.

L'utilisation d'adjuvants dans les compositions est connue depuis longtemps. Ces adjuvants ont pour objet principal de permettre l'augmentation de la réponse immunitaire. Ces adjuvants sont de natures diverses. Ils peuvent par exemple consister en des liposomes, des émulsions comprenant au moins une phase huile et au moins une phase aqueuse, du type adjuvants de Freund ou, de manière plus courante en des sels minéraux insolubles dans l'eau. Ces sels minéraux peuvent constituer par exemple en l'hydroxyde d'aluminium le nitrate de cérium, le sulfate de zinc, l'hydroxyde de fer colloïdal ou le chlorure de calcium. L'hydroxyde d'aluminium est l'adjuvant le plus couramment utilisé. Ces adjuvants sont décrits notamment dans l'article de Rajesh K. Gupta et al "Adjuvants, balance between toxicity and adjuvanticity", Vaccine, vol. 11, Issue 3, 1993, pages 993-306.

Les adjuvants mentionnés ci-dessus présentent l'inconvénient d'une efficacité faible. En outre, il est connu que l'hydroxyde d'aluminium n'induit efficacement qu'une immunité humorale, et non une immunité cellulaire. Par ailleurs, ils peuvent induire une certaine toxicité vis-à-vis des sujets traités. Plus particulièrement, lorsque ces compositions thérapeutiques sont injectées, on observe la formation de lésions et d'autres réactions locales telles que des granulomes au niveau du point d'injection.

Un premier objet de l'invention consiste en des compositions thérapeutiques comprenant un adjuvant permettant une augmentation de la réponse immune supérieure à celle conférée par l'hydroxyde d'aluminium, sans engendrer de lésions ou de réactions locales du type granulomes.

Selon un autre aspect, l'invention a pour objet une composition thérapeutique comprenant un adjuvant induisant efficacement aussi bien une immunité cellulaire qu'une immunité humorale.

L'invention a pour objet une composition comprenant :
(i) au moins un antigène ou au moins un générateur in vivo d'un composé comprenant une séquence d'acides aminés ; et,
(ii) au moins un adjuvant,
caractérisée en ce que ledit adjuvant contient au moins un sel pharmaceutiquement acceptable et soluble dans l'eau, constitué d'un anion organique ou d'un cation métallique trivalent.

Par antigène ou au moins un générateur in vivo d'un composé comprenant une séquence d'acides aminés, on désigne soit des micro-organismes tués, tels que les virus, les bactéries ou les parasites, soit des fractions purifiées de ces micro-organismes, soit des micro-organismes vivants dont le pouvoir pathogène a été atténué ; on peut citer notamment un virus recombinant notamment un virus non enveloppé tel que les adénovirus, le virus de la vaccine, le virus Canarypox, les herpès virus, les baculovirus ; on désigne aussi un vecteur recombinant viral non enveloppé vivant dont le génome contient, insérée de préférence dans une partie non essentielle pour la réplication du virus enveloppé correspondant, une séquence codant pour une sous-unité antigénique induisant une synthèse d'anticorps et/ou un effet protecteur contre le susdit virus enveloppé ou micro-organisme pathogène ; ces sous-unités antigéniques peuvent être par exemple, une protéine, une glycoprotéine, un peptide, ou une fraction peptidique et/ou protectrice contre une infection par un micro-organisme vivant tel un virus enveloppé, une bactérie ou un parasite.

On peut citer notamment un plasmide recombinant constitué d'une séquence de nucléotide dans laquelle est insérée une séquence nucléotidique exogène, provenant d'un micro-organisme ou d'un virus pathogène. Cette dernière séquence nucléotidique a pour but de permettre l'expression d'un composé comprenant une séquence d'acides aminés, ce composé ayant lui-même pour but de déclencher une réaction immune dans un organisme hôte ;

Un sel soluble dans l'eau au sens de la présente invention peut être tel que sa solubilité dans l'eau est supérieure ou égale à 10 g/l et, de préférence, comprise entre 10 et 2000 g/l. Le cation métallique trivalent constitutif dudit sel pharmaceutiquement acceptable est de préférence le cation trivalent de l'aluminium Al⁺⁺⁺. Il a en effet été constaté que ce sel pharmaceutiquement acceptable est soluble dans l'eau et permet l'induction d'une réponse immune particulièrement importante tout en présentant une toxicité faible. L'anion organique constitutif dudit sel pharmaceutiquement acceptable est avantageusement un anion d'un composé comprenant au moins un groupe fonctionnel oxygéné de préférence un groupe phosphorique ou un groupe carboxylique. L'acide glycérophosphorique est un anion à groupe phosphorique préféré. Les anions comprenant au moins un groupe carboxylique préféré sont dérivés de composés choisis parmi:
les oses acides de préférence des oses acides ayant de 5 à 7 atomes de carbone, plus préférentiellement ceux ayant 6 à 7 atomes de carbone, les acides mono ou polycarboxyliques, les acides aminés.

Les acides mono ou polycarboxyliques préférés sont l'acide fumarique, l'acide salicylique ou les composés de formule (I) 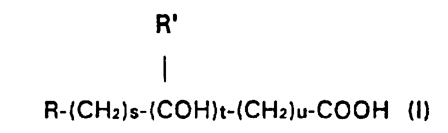 où R représente COOH, CH₃CO, CH₃ ou CH₂OH,
R' représente H ou COOH
et s, t et u, identiques ou différents sont compris entre 0 et 3.

Des composés de formule (I) préférés sont l'acide acétique, l'acide lactique, l'acide tartrique, l'acide malique, l'acide citrique, l'acide pyruvique.

Au sens de la présente invention, un ose acide consiste en un ose comprenant au moins une fonction carboxylique, un ose étant un glucide constitué par les sucres réducteurs. Ces oses acides sont avantageusement des dérivés d'aldoses, obtenus par oxydation de la fonction alcool primaire ou de la fonction aldéhydique en fonction carboxylique. De tels composés peuvent tout particulièrement consister en l'acide gluconique, l'acide glucuronique, l'acide fructoheptonique, l'acide gluconoheptonique, l'acide glucoheptonique. Lorsque l'anion organique est un dérivé d'un acide aminé, cet acide aminé peut être un acide α-aminé tel que l'acide glutamique, la méthionine et, tout particulièrement, l'acide aspartique.

Une composition ainsi définie contient entre 0,02 et 3000 mg/ml, de préférence 0, 1 et 1000 mg/ml du sel pharmaceutiquement acceptable et, notamment, entre 0,1 et 150 mg/ml.

L'invention a notamment pour objet la composition telle que définie précédemment dans laquelle l'adjuvant comprend le salicylate d'aluminium ou l'acétate d'aluminium soluble.

Dans une première variante de l'invention, la composition telle que définie ci-dessus peut également contenir une huile. Dans un tel cas, la composition selon l'invention se présente avantageusement sous la forme d'un émulsion associant au moins une phase aqueuse et au moins une phase huile. Cette émulsion peut être du type huile dans eau (H/E), eau dans huile (E/H), eau dans huile dans eau (E/HIE) ou microémulsion. On préfère cependant les émulsions E/H/E et H/E. Une telle émulsion peut être préparée selon les méthodes classiques de préparation d'une émulsion, notamment selon les procédés décrits dans les demandes de brevet EP-A-489 181 et EP-A-481 982. Ainsi, on peut émulsionner sous agitation l'huile constitutive de la phase huile avec la phase aqueuse constituée d'une solution ou d'une suspension aqueuse contenant l'antigène.

Une émulsion selon l'invention peut comporter, en poids, de 5 à 95 % de phase huile pour 95 à 5 % de phase aqueuse et, de préférence, de 25 à 75 % de phase huile pour 75 à 25 % de phase auqueuse. L'émulsion doit être stable de préférence pendant au moins 12 mois quand elle est stockée à 4°C. Les huiles, constituées de ladite phase huileuse pouvant être utilisées dans la composition selon l'invention sont avantageusement des huiles fluides choisies parmi les huiles minérales naturelles ou synthétiques, ou les huiles non minérales d'origine végétale, animale ou synthétique connues pour leur absence ou leur faible toxicité.

Elles doivent être liquides à la température de stockage (+4°C) ou au moins donner des émulsions liquides à cette température. On choisira en particulier des huiles minérales à chaîne linéaire ayant un nombre d'atomes de carbone de préférence supérieur à 16 et exempte de composés aromatiques. Des exemples connus sont le MARCOL 52 et le DRAKEOL 6VR. On peut également utiliser des huiles synthétiques telles que les polyisobutènes ou les polyisopropènes. Parmi les huiles végétales, on choisira les huiles insaturées riches en acide oléique qui sont biodégradables, par exemple les huiles d'arachide, d'olive, de sésame, de soja ou de germes de blé. Pour les huiles animales, les mêmes critères de tolérance et d'efficacité immunitaire sont requises. A titre d'exemple comme huiles animales, on peut citer le squalane ou l'huile de spermaceti. A titre d'huile non minérale synthétique, on peut encore citer les esters d'alcools et d'acides gras tels que, par exemple, l'oléate d'éthyle, le myristate d'isopropyle, les mono di ou triglycérides, les esters de propylène glycol, les glycérides partiels, tels que les glycérides d'huile de maïs comme ceux commercialisés sous le nom de LANOL par la Société SEPPIC, la maïsine, ou l'oléate d'oléyle. Par ailleurs, la composition selon l'invention quand elle se présente sous forme d'une émulsion telle que définie plus haut, peut également avantageusement comporter un ou plusieurs agents tensioactifs émulgateurs. Ce dernier présente un caractère lipophile ou hydrophile caractérisé par une valeur HLB (hydrophile-lipophile-balance) comprise entre 1 et 19.

Un tel tensioactif peut consister en
- un alkylpolyglycoside ou un mélange d'alkylpolyglycosides de formule Rₐ-(O)-Zn où Rₐ représente un radical aliphatique saturé, linéaire ou ramifié, comprenant de 4 à 24 atomes de carbone, Z est le reste d'un sucre, de préférence le glucose et n est compris entre 1 et 5 de préférence entre 1,1 et 2,
- les saponines,
- les lécithines,
- les alcanols polyoxyéthylés, tels que ceux commercialisés sous la désignation BRIJ par la société ICI,
- les polymères comprenant des blocs polyoxyéthylènes et polyoxypropylènes, tels que ceux commercialisés sous la désignation PLURONICS par la société BASF.

Des tensioactifs particulièrement préférés sont les esters de polyéthylèneglycol, obtenus par condensation d'un acide gras, notamment un acide gras liquide à 20°C avec un polyéthylèneglycol de poids moléculaire compris entre 80 et 200 ; un tel tensioactif est commercialisé par la Société SEPPIC sous la marque SIMULSOL 2599.

Un autre agent tensioactif préféré dans le cadre de la présente invention consiste en un ester obtenu par condensation d'un acide gras, avantageusement un acide gras liquide à 20°C avec un sucre, le sorbitol ou du glycérol. Ledit sucre peut consister en glucose, saccharose ou, de préférence, mannitol. A titre d'ester de mannitol particulièrement préféré, on peut citer des oléates de mannitol obtenus par anhydrisation de la chaîne carbonée polyhydroxylée du mannitol qui se cyclise en 1-4 ou en 2-6.

Des dérivés de ces esters de sucre de polyéthylèneglycol, de sorbitol ou de glycérol peuvent être également mis en oeuvre. Ces dérivés présentent une hydrophilie modifiée notamment par greffage de fonctions hydrophiles telles que alcool, polyol, oxyde d'éthylène, oxyde de propylène, acide carboxylique, amine ou amide. De tels dérivés peuvent par exemple consister en des esters gras de sorbitanne polyoxyéthylés, tels le TWEEN.

Les autres types de tensioactifs préférés consistent en des huiles végétales éthoxylés, telles que, par exemple l'huile de ricin polyéthoxylée, cette huile étant hydrogénée ou non, ou des esters de polyglycerol, notamment des esters de polyglycérol d'acides gras naturels tels que l'acide oléique, l'acide stéarique, l'acide ricinoléique ou l'acide isostéarique et, particulièrement, des ricinoléates ou des polyricinoléates de polyglycérol.

Un agent tensioactif selon l'invention est de préférence pharmaceutiquement acceptable au niveau des muqueuses ; il doit notamment être dépourvu de métaux lourds et présenter des indices d'acides ou de peroxydes très faibles. Il est également souhaitable qu'il satisfasse les normes de tests d'innocuité tels que par exemple, ceux décrits par S.S. Berllin, Annales of Allergy, 1962, 20, 473 ou les tests de toxicité anormale décrits dans la pharmacopée européenne. Préférentiellement, l'agent tensioactif est associé à l'adjuvant huileux avant formation de l'émulsion.

La concentration en agent tensioactif dans la composition telle que définie précédemment peut être comprise entre 0,01 et 500 mg/ml, et, de préférence, entre 0,1 et 200 mg/ml.

Des huiles associées avec un agent tensioactif (ester de mannitol) convenant tout particulièrement dans le cadre de la présente invention sont celles commercialisées par la Société SEPPIC sous la marque MONTANIDE. La nature de ces huiles, le type d'émulsion qu'elles permettent d'obtenir et les caractéristiques (viscosité et conductivité) de ces émulsions sont décrites dans la demande internationale WO94/16681, publiée le 4 août 1994 et, notamment, page 11, tableau I. Ces compositions selon l'invention peuvent être utilisées pour le traitement des maladies humaines ou vétérinaires. Elles permettent notamment de soulager les maladies infectieuses avec agent intracellulaire, notamment les maladies respiratoires, les maladies infectieuses opportunistes et dans le cadre de la médecine vétérinaire les maladies infectieuses des poissons.

La composition selon l'invention peut contenir aussi des substances modulatrices de l'immunité.

L'adjuvant huileux peut encore consister en une huile auto-émulsionnable, c'est-à-dire une préparation huileuse capable de former une émulsion stable avec une phase aqueuse, pratiquement sans apport d'énergie, par exemple par dispersion dans la phase aqueuse par agitation mécanique lente. A ce titre, on peut citer des huiles auto-émulsionnables telles connues dans la pharmacopée européenne sous les désignations Labrafil et Simulsol. Ces huiles sont des glycérides polyglycosés.

Des huiles auto-émulsionnables préférées sont celles décrites dans la demande de brevet français publiée sous le numéro 2 729 307, au nom de la Demanderesse, intitulée "utilisation d'esters d'acides gras éthoxylés comme composants auto-émulsionnables notamment utiles pour la préparation de compositions phytosanitaires ou de médicaments à usage vétérinaire ou humain", dont référence est intégrée à la présente description. Ces huiles consistent en des esters d'acides gras éthoxylés répondant à l'une des formules suivantes : 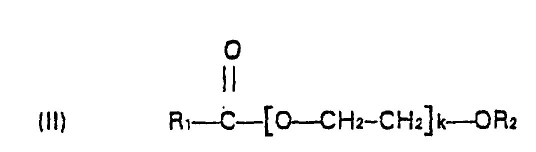 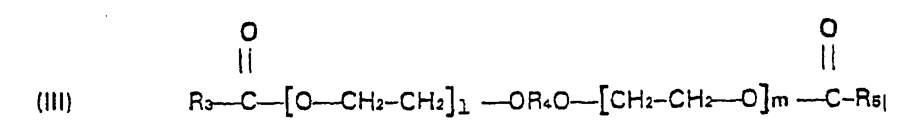 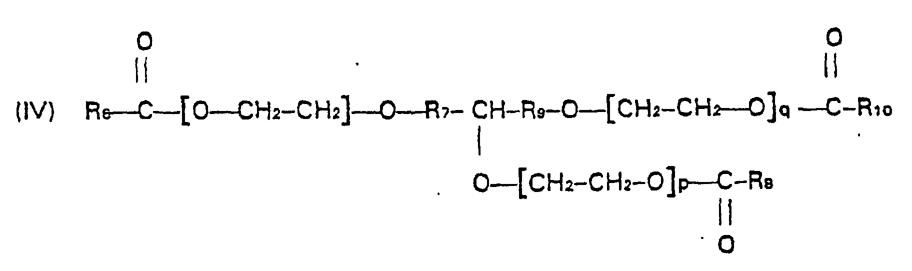 dans lesquelles
- R₁, R₃, R₅, R₆, R₈ et R₁₀ représentent une chaîne hydrocarbonée, linéaire ou ramifiée, saturée ou insaturée ayant de 5 à 30 atomes de carbone ;
- R₂, R₄, R₇ et R₉ représentent une chaîne hydrocarbonée, linéaire ou ramifiée, saturée ou insaturée ayant de 1 à 5 atomes de carbone ;
   le nombre total de molécules d'oxyde d'éthylène respectivement représenté dans les formules II, III et IV précitées par k, l+m, n+p+q étant un nombre entier tel que la valeur HLB (balance hydrophile-lipophile) desdits composés soit comprise entre environ 4 et environ 10, de préférence entre environ 5 et environ 9.
   R₁ est de préférence choisi parmi les restes des acides palmitique, stéarique, ricinoléique, oléique, linoléique et linolénique et R₂ représente un radical méthyle et k est un nombre entier compris entre 1 et 5, de préférence égal à 2, et par ailleurs, les esters d'acides gras éthoxylés de formule III préférés sont ceux où :
   (i) - R₆, R₈ et R₁₀ représentent des chaînes hydrocarbonées ayant de 16 à 22 atomes de carbone correspondant notamment aux chaînes grasses de l'huile de colza, de maïs, de soja, d'arachide et de noyaux d'abricots ;
- R₇ et R₉ représentent un groupe méthylène CH₂ ;
- n, p, q représentent des nombres entiers tels que leur somme soit comprise entre 3 et 30, et de préférence égale à 20 ; ou
   (ii) - R₆, R₈ et R₁₀ représentent des chaînes hydrocarbonées correspondant aux chaînes grasses de l'huile de ricin ;
- R₇ et R₉ représentent un radical méthylène CH₂ ;
- n, p, q représentent des nombres entiers tels que leur somme soit comprise entre 5 et 7.

La concentration en huile auto-émulsionnable dans la composition selon l'invention peut être comprise entre environ 5 et 700 g/l, de préférence entre environ 10 et 500 g/l.

Outre la phase huile et la phase aqueuse, la composition selon l'invention peut comporter un agent stimulant immunitaire conventionnel tel l'Avridine®, soit la N,N-dioctadecyl-N',N'-bis(2-hydroxyéthyl) propane-diamine, les dérivés du MDP (muramyl dipeptide) notamment le threonyl-MDP, les dérivés de l'acide mycolique ou les dérivés du Lipide A.

La composition selon l'invention peut comprendre encore un ou plusieurs agents tensioactifs, en l'absence de tout adjuvant huileux.

La composition se présente alors sous la forme d'une solution micellaire. Celle-ci peut être préparée par simple mélange de l'agent tensioactif avec une dispersion dans l'eau de l'antigène ou du générateur d'antigène in vivo.

L'agent tensioactif peut être choisi parmi les agents tensioactifs décrits plus haut, en association avec un adjuvant huileux.

Ladite solution micellaire peut comporter de 0,5 à 500 mg/ml, de préférence de 1 à 250 mg/ml en agent tensioactif.

Une composition selon l'invention peut aussi comporter un composé sympathomimétrique.

Par composés sympathomimétiques, on désigne notamment les amphétamines, les catécholamines, les phénylisopropylamines ou la tyramine. Comme exemples de tels composés, on peut citer notamment l'isoprotérénol, la L-Epinephrine, le lévartérénol, l'éphédrine, la phényléphédrine ou le salbutamol.

Une composition selon la présente invention peut comprendre un antigène tel qu'un virus, un micro-organisme, plus particulièrement une bactérie ou un parasite, ou un composé comprenant une chaîne peptidique. Un tel composé peut consister une protéine ou une glycoprotéine, notamment une protéine ou une glycoprotéine issues d'un micro-organisme, un peptide synthétique ou une protéine ou un peptide issu du génie génétique. Lesdits virus et micro-organisme peuvent être totalement inactivés ou vivants atténués. A titre de virus pouvant constituer un antigène selon la présente invention, on peut citer le virus de la rage, les herpès virus tels que le virus de la maladie d'Aujeszky, les orthomixovirus tels que Influenzae, les picornavirus tels que le virus de la fièvre aphteuse ou les rétrovirus tels que les VIH. A titre de micro-organisme du type bactérien pouvant constituer un antigène selon la présente invention, on peut citer E. coli, et ceux des genres Pasteurella, Furonculosis, Vibriosis, Staphylococcus et Streptococcus. A titre de parasite, on peut citer ceux des genres Trypanosoma, Plasmodium et Leishmania.

Une composition selon l'invention comprend une concentration en antigène qui dépend de la nature de cet antigène et de la nature du sujet traité. Il est toutefois particulièrement remarquable qu'un adjuvant selon l'invention, associé ou non à un adjuvant huileux et/ou un agent tensioactif tels que définis plus haut, permet de diminuer d'une façon notable la dose habituelle d'antigène requise. La concentration adéquate d'antigène peut être déterminée de manière classique par l'homme du métier. Généralement, cette dose est de l'ordre de 0,1µg/ml à 1g/ml plus généralement comprise entre 1 µg/ml et 100mg/ml.

Une composition selon l'invention peut aussi comprendre un générateur in vivo d'un composé comprenant une séquence d'acides aminés, c'est-à-dire un composé biologique capable d'exprimer un tel composé dans l'organisme hôte dans lequel on a introduit ledit générateur in vivo. Le composé comprenant la séquence d'acides aminés, peut être une protéine, un peptide ou une glycoprotéine.

Ces générateurs in vivo sont généralement obtenus par des procédés issus du génie génétique.

Plus particulièrement, ils peuvent consister en des micro-organismes vivants, généralement un virus, jouant le rôle de vecteur recombinant, dans lequel est insérée une séquence nucléotidique, notamment un gène exogène. Ces composés sont connus en tant que tels et utilisés notamment comme vaccin sous unitaire recombinant.

A cet égard, on peut se référer à l'Article de M. ELOIT et al., Journal of virology (1990) 71, 2925-2431 dans la demande internationale WO-A-91/00107 ou à la demande internationale WO-A-94/16681.

Avantageusement, le micro-organisme constitutif d'un vaccin sous-unitaire recombinant est un virus recombinant non enveloppé, par exemple choisi parmi les adénovirus, le virus de la vaccine, le canarypox virus, les herpès virus ou les baculovirus. Le gène exogène inséré dans le micro-organisme peut être, par exemple, issu d'un virus Aujeszky ou HIV.

Les générateurs in vivo selon l'invention peuvent aussi consister en un plasmide recombinant comprenant une séquence nucléotidique exogène, capable d'exprimer dans un organisme hôte un composé comprenant une séquence d'acides aminés. De tels plasmides recombinants et leur mode d'administration dans un organisme hôte ont été décrits en 1990, par LIN et al., Circulation 82:2217,2221 ; COX et al., J. of VIROL, Sept. 1993, 67, 9, 5664-5667 et dans la demande internationale WO/FR 95/00345 du 21 Mars 1995, au nom de la Demanderesse, intitulée "Une composition comprenant un plasmide recombinant et ses utilisations comme vaccin et médicament".

Selon la nature de la séquence nucléotidique comprise dans le générateur in vivo, le composé comprenant la séquence d'acides aminés qui est exprimé au sein de l'organisme hôte, peut :
(i) être un antigène, et permettre le déclenchement d'une réaction immune,
(ii) avoir une action curative vis-à-vis d'une maladie essentiellement une maladie d'ordre fonctionnel, qui s'est déclenchée chez l'organisme hôte. Dans ce cas, le générateur in vivo permet un traitement de l'hôte, du type thérapie génique.

A titre d'exemple, une telle action curative peut consister en une synthèse par le générateur in vivo de cytokine, comme les interleukines, notamment l'interleukine 2. Celles-ci permettent le déclenchement ou le renforcement d'une réaction immune visant à l'élimination sélective des cellules cancéreuses.

La concentration en ledit générateur in vivo dans la composition selon l'invention dépend, là encore, notamment de la nature dudit générateur et de l'hôte dans lequel il est administré. Cette concentration peut être aisément déterminée par l'homme du métier, sur la base d'expérience de routine.

A titre indicatif, on peut toutefois préciser que lorsque le générateur in vivo est un microorganisme recombinant, sa concentration dans la composition selon l'invention peut être comprisé entre 10² et 10¹⁵ micro-organismes/ml, de préférence entre 10⁵ et 10¹² micro-organismes/ml.

Lorsque le générateur in vivo est un plasmide recombinant, sa concentration dans la composition selon l'invention peut être comprise entre 0,01 et 100 g/l.

Une composition selon l'invention peut être utilisée comme médicament préventif ou curatif. Selon la nature de l'antigène ou du générateur in vivo, une composition selon l'invention peut être administrée à des poissons, des crustacés tels que les crevettes, des volailles, notamment, des oies, des dindes, des pigeons et des poulets, aux canidés tels le chien, aux félidés tels le chat, aux porcs, aux primates, aux bovidés, aux ovidés et aux chevaux, pour traiter par exemple la toxoplasmose ovine ou le pou du saumon. La composition selon l'invention peut être également administrée à l'homme. L'administration de la composition peut se faire de manière classique par voie parentérale, notamment par injection sous-cutanée, intramusculaire ou intrapéritonéale ou par voie mucosale notamment par voie orale, voie rectale, voie nasale, voie vaginale.

Selon un autre aspect de l'invention, celle-ci consiste en l'utilisation d'un adjuvant tel que défini ci-dessus pour la préparation d'un vaccin destiné à la prévention ou au traitement d'une maladie infectieuse, notamment une maladie infectieuse engendrée par un virus ou un micro-organisme tels ceux mentionnés plus haut.

Selon un autre aspect de l'invention, celle-ci consiste en l'utilisation de cet adjuvant pour la préparation d'une composition destinée à soigner une maladie d'ordre fonctionnel, telle le cancer ou la mucoviscidose.

Dans l'une ou l'autre de ces utilisations, ledit adjuvant peut être associé à un adjuvant huileux, un agent tensioactif ou à un adjuvant huileux lui-même associé à un agent tensioactif, ces adjuvants huileux et tensioactifs étant tels que définis plus haut.

Des compositions adjuvantes comprenant ledit sel pharmaceutiquement acceptable et l'adjuvant huileux et/ou les tensioactifs précités et, éventuellement, ledit composé sympathomimétique constituent encore un autre aspect de l'invention. Le cas échéant, ces compositions adjuvantes comprennent une phase aqueuse.

Dans ce dernier cas, les compositions adjuvantes selon l'invention, comprenant au moins un adjuvant huileux, et, le cas échéant, un tensioactif, peuvent se présenter sous la forme d'une émulsion. Cette dernière peut être du type E/H, H/E, E/H/E ou microémulsion.

Ces émulsions peuvent comprendre en poids, de 0,5 % à 99,5 % de phase huile pour 99,5 % à 0,5 % de phase aqueuse, de préférence, de 5 à 95 % de phase huile pour 95 à 5 % de phase aqueuse et, plus préférentiellement, de 25 à 75 % de phase huile pour 75 à 25 % de phase aqueuse.

Le cas échéant, elles peuvent comprendre de 0,01 à 500 mg/ml, de préférence de 0,1 à 200 mg/ml d'un tensioactif au moins.

Lorsque la composition adjuvante selon l'invention ne comprend, outre le sel pharmaceutiquement acceptable, le composé sympathomimétique, et une phase aqueuse, qu'un ou plusieurs tensioactifs, elle se présente alors sous la forme d'une solution micellaire. La teneur en tensioactif de cette solution micellaire peut être comprise entre 0,01 et 900 mg/ml, de préférence entre 1 et 250 mg/ml.

Une composition adjuvante selon l'invention comprend habituellement le sel pharmaceutiquement acceptable tel que défini précédemment à une concentration de 0,02 à 3000 mg/ml, de préférence 0,1 à 1000 mg/ml, plus préférentiellement de 0,1 à 150 mg/ml, le composé sympathomimétique c'est à une concentration de 10⁻¹⁰ molaire à 10⁻² molaire de préférence de 10⁻⁷ Molaire à 10⁻⁵ Molaire.

Ces compositions adjuvantes sont utiles pour préparer les compositions selon l'invention.

Ces dernières peuvent alors être préparées par simple mélange de la composition adjuvante avec une composition comprenant un antigène ou un générateur in vivo d'un composé comprenant une séquence d'acides aminés.

Les exemples suivants permet de mettre en évidence les propriétés des compositions selon la présente invention.

### Exemple 1 :

On a comparé l'effet immunostimulant de 2 compositions comprenant des sels solubles selon l'invention, acétate d'Aluminium soluble (C₂H₅AlO₄) et le salicylate d'Aluminium, avec celui de sels insolubles, comme l'hydroxyde d'Aluminium, le phosphate d'Aluminium et une forme insoluble d'acétate d'Aluminium (C₄H₇AlO₅). Ces compositions thérapeutiques comprennent, à titre d'antigène de la sérum albumine bovine (BSA), Grade V, Sigma.

Ces compositions thérapeutiques (dose/souris) d'un volume de 100 µl mises en oeuvre comprenaient 10µg d'antigène et ont été injectées par voie sous-cutanée.

Les concentrations des sels testés correspondent aux concentrations optimales d'utilisation.

Les résultats ont été obtenus 21 jours après vaccination:

| **Adjuvant** | **réponse lgG1** | **réponse lgG2a** |
|---|---|---|
| - | <1000 | <1000 |
| C₂H₅AlO₄ (forme soluble) | 64000 | 8000 |
| Salicylate d'Aluminium | 48000 | 6000 |
| AlOH | 8000 | <1000 |
| C₄H₇AlO₅ (forme insoluble) | <1000 | <1000 |
| AlPO₄ | <1000 | <1000 |

Les réponses IgG 1 sont représentatives de la réponse humorale,

Les réponses IgG 2a sont représentatives de la réponse cellulaire.

Les réponses humorales et cellulaires obtenues avec un adjuvant selon l'invention sont significativement supérieures à celles obtenues avec un adjuvant conventionnel tel Al(OH)₃, ou AlPO₄.

D'autre part, on met clairement en évidence l'intérêt d'utiliser des adjuvants solubles dans l'eau par rapport à l'utilisation de forme insoluble dans l'eau.

### Exemple 2:

On a injecté à différents lots comprenant chacun 5 souris, une composition thérapeutique d'un volume de 1000 µl, comprenant 10 µg d'antigène (BSA), par voie sous-cutanée. On a évalué les réactions locales (lésions et granulomes) 8 jours après injection.

| | souris 1 | souris 2 | souris 3 | souris 4 | souris 5 |
|---|---|---|---|---|---|
| C₂H₅AlO₄ (forme soluble) | 0 | 0 | 0 | + | 0 |
| Salicylate d'Aluminium | 0 | + | 0 | 0 | 0 |
| AlOH | ++ | +++ | ++ | +++ | ++ |

Les réactions locales obtenues avec un adjuvant selon l'invention sont significativement inférieures à celles obtenues avec un adjuvant conventionnel tel Al(OH)₃.

## Revendications

1. Composition comprenant :
(i) au moins un antigène ou au moins un générateur in vivo d'un composé comprenant une séquence d'acides aminés; et,
(ii) au moins un adjuvant,
**caractérisée en ce que** ledit adjuvant contient au moins un sel pharmaceutiquement acceptable et soluble dans l'eau constitué d'un anion organique et d'un cation métallique trivalent.

2. Composition selon la revendication 1, **caractérisée en ce que** le cation métallique trivalent est le cation Al⁺⁺⁺.

3. Composition selon la revendication 2, **caractérisée en ce que** l'anion organique est dérivé d'un composé comprenant au moins un groupe fonctionnel oxygéné tel que de préférence un groupe carboxylique ou un groupe phosphorique.

4. Composition selon la revendication 3, **caractérisée en ce que** l'anion organique est dérivé d'un composé choisi parmi l'acide glycérophosphorique, l'acide salicylique, l'acide acétique, l'acide aspartique, l'acide gluconique, l'acide fructoheptonique et l'acide glucoheptonique.

5. Composition selon la revendication 4, **caractérisée en ce que** le sel pharmaceutiquement acceptable est le salicylate d'aluminium ou l'acétate d'aluminium solubles.

6. Composition selon l'une des revendications 1 à 5, **caractérisée en ce qu'**elle se présente sous la forme d'une émulsion comprenant au moins une phase aqueuse et au moins une phase huile, la phase huile étant constituée par un adjuvant, et l'émulsion de préférence du type E/H/E, H/E ou microémulsion.

7. Composition selon la revendication 6, **caractérisée en ce que** l'adjuvant huileux est associé à un agent tensioactif, de préférence un agent tensioactif choisi soit parmi un ester obtenu par condensation d'un acide gras avec un sucre, un polyéthylèneglycol, le sorbitol ou le glycérol, ou un dérivé d'un tel ester dont l'hydrophilie a été modifiée, une huile végétale éthoxylée ou leurs mélanges, soit parmi les esters de polyglycérol, notamment un ester de polyglycérol d'acides gras naturels et, de préférence, les ricinoléates ou les polyricinoléates de polyglycérol ou constituant en des huiles de ricin polyéthoxylées hydrogénées ou non.

8. Composition selon la revendication 6, **caractérisée en ce que** l'adjuvant huileux est une huile auto-émulsionnable, notamment. un ester d'acides gras éthoxylés répondant à l'une des formules suivantes : 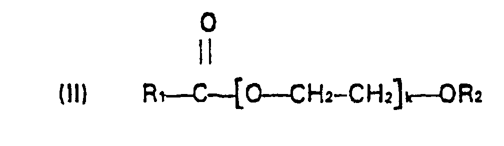 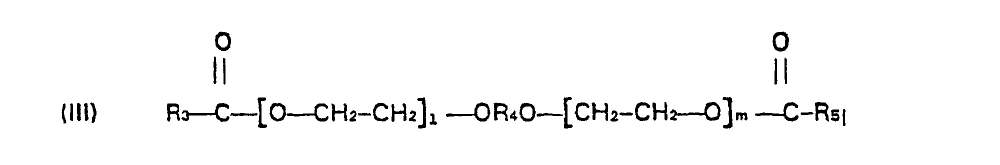 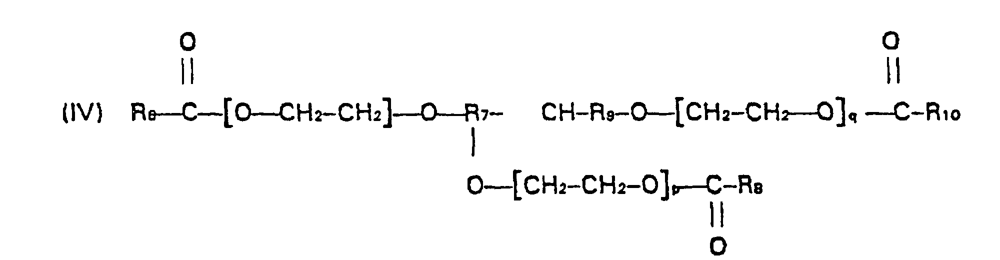 dans lesquelles :
- R₁, R₃, R₅, R₆, R₈ et R₁₀ représentent une chaîne hydrocarbonée, linéaire ou ramifiée, saturée ou insaturée ayant de 5 à 30 atomes de carbone ;
- R₂, R₄, R₇ et R₉ représentent une chaîne hydrocarbonée, linéaire ou ramifiée, saturée ou insaturée ayant de 1 à 5 atomes de carbone ;
le nombre total de molécules d'oxyde d'éthylène respectivement représenté dans les formules II, III et IV précitées par k, l+m, n+p+q étant un nombre entier tel que la valeur HLB (balance hydrophile-lipophile) desdits composés soit comprise entre environ 4 et environ 10, de préférence entre environ 5 et environ 9.

9. Composition selon l'une des revendications 1 à 5, **caractérisée en ce qu'**elle comprend en outre un agent tensioactif, ladite composition se présentant sous la forme d'une solution micellaire.

10. Composition selon la revendication 9, **caractérisée en ce que** l'agent tensioactif est soit un ester obtenu par condensation d'un acide gras avec un sucre ou du glycérol, ou un dérivé d'un tel ester dont l'hydrophilie a été modifiée ou soit une huile végétale éthoxylée.

11. Composition selon l'une des revendications 1 à 10, comprenant en outre un stimulant immunitaire tel que l'Avridine®, les dérivés du muramyl dipeptide, l'acide mycolique ou les dérivés du lipide A.

12. Composition selon l'une des revendications 1 à 11 comportant en outre un composé sympathomimétique est choisi parmi les sympathomimétiques amines telles qu'une catécholamine, une amphétamine, une phénylisopropylamine ou la tyramine et, de préférence, choisi parmi l'éphédrine ou l'isoprotérénol, la L-Epinephrine, le lévartérénol, l'éphédrine, la phényléphédrine ou le salbutamol.

13. Composition selon l'une des revendications 1 à 12, pour la mise en oeuvre d'une méthode de traitement du corps humain ou animal.

14. Médicament selon la revendication 13 destiné à traiter ou à prévenir les maladies infectieuses et/ou fonctionnelles et notamment les maladies engendrées par un virus, un microorganisme ou un parasite telles que les maladies respiratoires.

15. Médicament selon la revendication 13 destiné à traiter la maladie d'Aujeszky chez les porcs, la toxoplasmose ovine ainsi que les maladies des poissons tel que le pou du saumon ou les maladies des crustacés.

16. Utilisation de l'adjuvant tel que défini à l'une des revendications 1 à 10 pour la fabrication de vaccins destinés à la vaccination muqueuse, notamment orale, nasale, rectale ou vaginale.

17. Composition adjuvante comprenant une phase aqueuse, un adjuvant huileux, un tensioactif, éventuellement un composé sympathomimétrique, **caractérisée en ce qu'**elle contient au moins un sel constitué d'un anion organique et d'un cation métallique trivalent et, notamment, le salicylate d'aluminium ou l'acétate d'aluminium soluble.

18. Composition selon la revendication 17 sous forme d'un émulsion E/H/E ou H/E.

## Claims

1. Composition comprising:
(i) at least one antigen or at least one in vivo generator of a compound comprising an amino acid sequence; and
(ii) at least one adjuvant,
**characterized in that** the said adjuvant contains at least one pharmaceutically acceptable and water-soluble salt consisting of an organic anion and a trivalent metal cation.

2. Composition according to Claim 1, **characterized in that** the trivalent metal cation is the Al⁺⁺⁺ cation.

3. Composition according to Claim 2, **characterized in that** the organic anion is derived from a compound comprising at least one oxygenated functional group, preferably such as a carboxylic group or a phosphoric group.

4. Composition according to Claim 3, **characterized in that** the organic anion is derived from a compound chosen from glycerophosphoric acid, salicylic acid, acetic acid, aspartic acid, gluconic acid, fructoheptonic acid and glucoheptonic acid.

5. Composition according to Claim 4, **characterized in that** the pharmaceutically acceptable salt is soluble aluminium salicylate or soluble aluminium acetate.

6. Composition according to one of Claims 1 to 5, **characterized in that** it is in the form of an emulsion comprising at least one aqueous phase and at least one oil phase, the oil phase consisting of an adjuvant, and the emulsion preferably being of the W/O/W, O/W or microemulsion type.

7. Composition according to Claim 6, **characterized in that** the oily adjuvant is combined with a surfactant, preferably a surfactant chosen from an ester obtained by condensation of a fatty acid with a sugar, a polyethylene glycol, sorbitol or glycerol, or a derivative of such an ester whose hydrophilicity has been modified, an ethoxylated plant oil or mixtures thereof, or from polyglycerol esters, in particular a polyglycerol ester of natural fatty acids and, preferably, polyglyceryl ricinoleates or polyricinoleates or an ester consisting of polyethoxylated castor oils which are optionally hydrogenated.

8. Composition according to Claim 6, **characterized in that** the oily adjuvant is a self-emulsifying oil, in particular an ester of ethoxylated fatty acids corresponding to one of the following formulae: 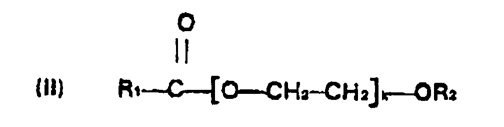 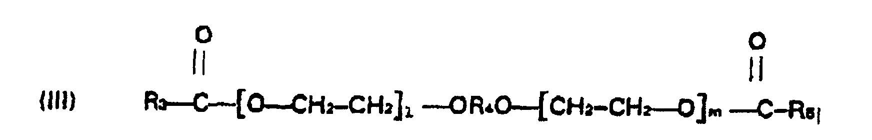 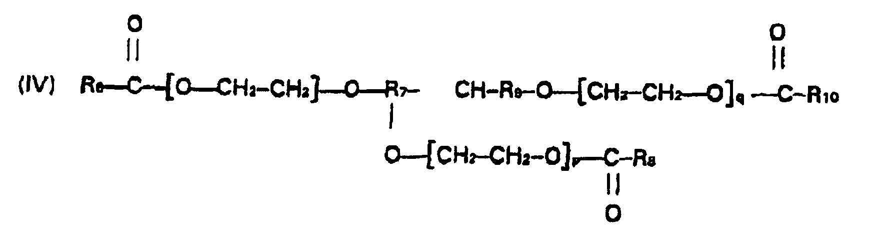 in which:
- R₁, R₃, R₅, R₆, R₈ and R₁₀ represent a saturated or unsaturated, linear or branched hydrocarbon-based chain containing from 5 to 30 carbon atoms;
- R₂, R₄, R₇ and R₉ represent a saturated or unsaturated, linear or branched hydrocarbon-based chain containing from 1 to 5 carbon atoms;
the total number of ethylene oxide molecules represented in the abovementioned formulae II, III and IV, respectively, by k, l+m, n+p+q being an integer such that the HLB (hydrophilic-lipophilic balance) value of the said compounds is between about 4 and about 10, preferably between about 5 and about 9.

9. Composition according to one of Claims 1 to 5, **characterized in that** it also comprises a surfactant, the said composition being in the form of a micellar solution.

10. Composition according to Claim 9, **characterized in that** the surfactant is either an ester obtained by condensation of a fatty acid with a sugar or glycerol, or a derivative of such an ester whose hydrophilicity has been modified, or is an ethoxylated plant oil.

11. Composition according to one of Claims 1 to 10, also comprising an immunostimulatory agent such as Avridine®, muramyl dipeptide derivatives, mycolic acid or lipid A derivatives.

12. Composition according to one of Claims 1 to 11, also containing a sympathomimetic compound chosen from sympathomimetic amines such as a catecholamine, an amphetamine, a phenylisopropylamine and tyramine, and preferably chosen from ephedrine or isoproterenol, L-epinephrine, levarterenol, ephedrine, phenylephedrine and salbutamol.

13. Composition according to one of Claims 1 to 12, for carrying out a method for treating the human or animal body.

14. Medicinal product according to Claim 13, which is intended to treat or prevent infectious and/or functional diseases and in particular diseases generated by a virus, a microorganism or a parasite, such as respiratory diseases.

15. Medicinal product according to Claim 13, which is intended to treat Aujeszky disease in pigs, ovine toxoplasmosis and diseases in fish, such as salmonpox or diseases in crustaceans.

16. Use of the adjuvant as defined in one of Claims 1 to 10 for the manufacture of vaccines intended for mucous vaccination, in particular oral, nasal, rectal or vaginal vaccination.

17. Adjuvant composition comprising an aqueous phase, an oily adjuvant, a surfactant and optionally a sympathomimetic compound, **characterized in that** it contains at least one salt consisting of an organic anion and a trivalent metal cation, and in particular soluble aluminium salicylate or soluble aluminium acetate.

18. Composition according to Claim 17, in the form of a W/O/W or O/W emulsion.

## Patentansprüche

1. Zusammensetzung mit:
(i) mindestens einem Antigen oder mindestens einem Objekt, das in vivo eine aus Aminosäuresequenzen bestehende Verbindung erzeugt, sowie
(ii) mindestens einem Adjuvans,
**dadurch gekennzeichnet, daß** das Adjuvans mindestens ein pharmazeutisch unbedenkliches, wasserlösliches Salz aus einem organischen Anion und einem dreiwertigen Metallkation enthält.

2. Zusammensetzung nach Anspruch 1, **dadurch gekennzeichnet, daß** es sich bei dem dreiwertigen Metallkation um das Kation Al⁺⁺⁺ handelt.

3. Zusammensetzung nach Anspruch 2, **dadurch gekennzeichnet, daß** das organische Anion von einer Verbindung mit mindestens einer sauerstoffhaltigen funktionellen Gruppe, wie vorzugsweise einer Carbonsäuregruppe oder einer Phosphorsäuregruppe, stammt.

4. Verbindung nach Anspruch 3, **dadurch gekennzeichnet, daß** das organische Anion von einer Verbindung aus der Gruppe Glycerinphosphorsäure, Salicylsäure, Essigsäure, Asparaginsäue, Gluconsäure, Fructoheptonsäure und Glucoheptonsäure stammt.

5. Verbindung nach Anspruch 4, **dadurch gekennzeichnet, daß** es sich bei dem pharmazeutisch unbedenklichen Salz um lösliches Aluminiumsalicylat oder lösliches Aluminiumacetat handelt.

6. Zusammensetzung nach einem der Ansprüche 1 bis 5, **dadurch gekennzeichnet, daß** sie in Form einer Emulsion mit mindestens einer wäßrigen Phase und mindestens einer öligen Phase vorliegt, wobei die ölige Phase aus einem Adjuvans besteht und die Emulsion vorzugsweise aus dem W/O/W-, O/W- oder Mikroemulsionstyp besteht.

7. Zusammensetzung nach Anspruch 6, **dadurch gekennzeichnet, daß** das ölige Adjuvans mit einem Tensid kombiniert ist, vorzugsweise mit einem Tensid aus der Gruppe durch Kondensation einer Fettsäure mit einem Zucker, einem Polyethylenglycol, Sorbit oder Glycerin erhaltener Ester bzw. ein Derivat solch eines Esters mit modifizierter Hydrophilie, ethoxylierter pflanzlicher Öle oder ihrer Mischungen, oder auch aus der Gruppe der Polyglycerinester, insbesondere ein Polyglycerinester von natürlichen Fettsäuren, vorzugsweise die Ricinoleate oder Polyricinoleate von Polyglycerin, oder gegebenenfalls hydrierte polyethoxylierte Ricinusöle enthält.

8. Zusammensetzung nach Anspruch 6, **dadurch gekennzeichnet, daß** es sich bei dem öligen Adjuvans um ein selbstemulgierbares Öl handelt, insbesondere einen Ester ethoxylierter Fettsäuren, der einer der folgenden Formeln entspricht: 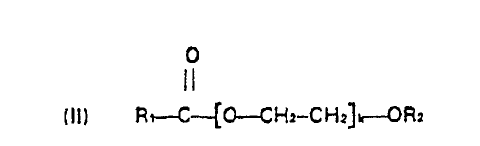 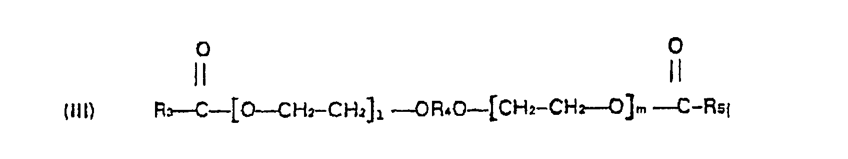 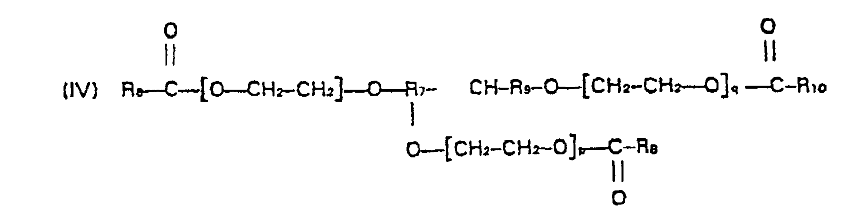 in denen:
R₁, R₃, R₅, R₆, R₈ und R₁₀ eine geradkettige oder verzweigte gesättigte oder ungesättigte Kohlenwasserstoffkette mit 5 bis 30 Kohlenstoffatomen darstellen und
R₂, R₄, R₇ und R₉ eine geradkettige oder verzweigte, gesättigte oder ungesättigte Kohlenwasserstoffkette mit 1 bis 5 Kohlenstoffatomen darstellen,
wobei die Gesamtzahl der durch k, l + m bzw. n + p + q in den oben genannten Formeln II, III bzw. IV dargestellten Ethylenoxidmoleküle eine ganze Zahl bedeutet, so daß der HLB-Wert (HLB: hydrophilic-lipophilic balance) dieser Verbindungen zwischen ungefähr 4 und ungefähr 10, vorzugsweise zwischen ungefähr 5 und ungefähr 9, beträgt.

9. Zusammensetzung nach einem der Ansprüche 1 bis 5, **dadurch gekennzeichnet, daß** diese weiterhin ein Tensid enthält, wobei die Zusammensetzung in Form einer Mizellenlösung vorliegt.

10. Zusammensetzung nach Anspruch 9, **dadurch gekennzeichnet, daß** es sich bei dem Tensid entweder um einen durch Kondensation einer Fettsäure mit einem Zucker oder Glycerin erhaltenen Ester bzw. ein Derivat solch eines Esters, dessen Hydrophilie modifiziert worden ist, oder um ein ethoxyliertes pflanzliches Öl handelt.

11. Zusammensetzung nach einem der Ansprüche 1 bis 10, die weiterhin ein Immunstimulanz wie Avridin® , Muramyldipeptidderivate, Mycolsäure oder Lipid-A-Derivate enthält.

12. Zusammensetzung nach einem der Ansprüche 1 bis 11 mit weiterhin einem Sympathomimetikum aus der Gruppe der sympathomimetisch wirksamen Amine wie ein Catecholamin, Amphetamin, Phenylisopropylamin oder das Tyramin, vorzugsweise aus der Gruppe Ephedrin oder Isoproterenol, L-Epinephrin, Levarterenol, Ephedrin, Phenylephedrin oder Salbutamol.

13. Zusammensetzung nach einem der Ansprüche 1 bis 12 zur Durchführung einer Methode zur Behandlung des menschlichen oder tierischen Körpers.

14. Arzneimittel nach Anspruch 13 zur Behandlung oder Vorbeugung von Infektionskrankheiten bzw. Funktionsstörungen, insbesondere Krankheiten, die von einem Virus, Mikroorganismus oder Parasiten hervorgerufen werden, wie Krankheiten der Atemwege.

15. Arzneimittel nach Anspruch 13 zur Behandlung von Aujeszky-Krankheit des Schweins, Toxoplasmose beim Schaf sowie Fischkrankheiten wie die Fischlaus beim Lachs oder Krankheiten von Krustentieren.

16. Verwendung eines nach einem der Ansprüche 1 bis 10 definierten Adjuvans zur Herstellung von Impfstoffen, die über die Schleimhaut verabreicht werden, insbesondere zur Oral-, Nasal-, Rektal- oder Vaginalimpfung.

17. Adjuvanszusammensetzung mit einer wäßrigen Phase, einem öligen Adjuvans, einem Tensid sowie gegebenenfalls einen Sympathomimetikum, **dadurch gekennzeichnet, daß** sie mindestens ein aus einem organischen Anion und einem dreiwertigen Metallkation bestehendes Salz, insbesondere lösliches Aluminiumacetat oder Aluminiumsalicylat, enthält.

18. Zusammensetzung nach Anspruch 17 in Form einer W/O/W- oder O/W-Emulsion.
